# EUROPÄISCHE PATENTSCHRIFT

(11) **EP 0 643 766 B1**
(45) Veröffentlichungstag und Bekanntmachung des Hinweises auf die Patenterteilung: **26.01.2000**
(21) Anmeldenummer: 93912797.3
(22) Anmeldetag: 26.05.1993
(51) Int. Cl.: C12N 1/26, C02F 1/68, C02F 3/34

(54) **VERBESSERTE NÄHRSTOFFGEMISCHE FÜR DIE BIOREMEDIATION VERSCHMUTZTER BÖDEN UND GEWÄSSER**
IMPROVED MIXTURE OF NUTRIENTS FOR THE BIOREMEDIATION OF POLLUTED SOILS AND WATERS
MELANGE AMELIORE DE SUBSTANCES NUTRITIVES UTILE POUR LA BIORESTAURATION DE SOLS ET D'EAUX CONTAMINES

(30) Priorität: 03.06.1992 DE 4218243
(43) Veröffentlichungstag der Anmeldung: 22.03.1995
(73) Patentinhaber: Cognis Deutschland GmbH, 40589 Düsseldorf (DE)
(72) Erfinder: KOPP-HOLTWIESCHE, Bettina, D-40599 Düsseldorf (DE); WEISS, Albrecht, D-40764 Langenfeld-Richrath (DE); BÖHME, Adelheid, D-40472 Düsseldorf (DE)
(74) Vertreter: Wilk, Hans-Christof, Dr.
(86) Internationale Anmeldenummer: EP9301323
(87) Internationale Veröffentlichungsnummer: WO9324612

(56) Entgegenhaltungen:
- DE-A- 2 739 428
- GB-A- 2 065 631
- GB-A- 2 115 399
- US-A- 4 276 094
- US-A- 4 462 910
- WATER ENVIRONMENT RESEARCH Bd. 64, Nr. 2, April 1992, ALEXANDRIA, VA US Seiten 163 - 169 , XP000296897 D. M. FALATKO ET AL 'EFFECTS OF BIOLOGICALLY PRODUCED SURFACTANTS ON THE MOBILITY AND BIODEGRADATION OF PETROLEUM HYDROCARBONS'

## Beschreibung

Die Bekämpfung von Verschmutzungen auf Basis von Kohlenwasserstoffverbindungen - beispielsweise Erdölverschmutzungen - in Böden und Gewässern mittels Bioremediation gewinnt zunehmende Bedeutung. Große Vorteile haben die kostengünstigen in situ-Verfahren, bei denen kein Deponieraum verbraucht wird. Kohlenwasserstoffverbindungen verzehrende Mikroorganismen sind im Rahmen dieser Technologie wertvolle Arbeitsmittel selbst unter vergleichsweise ungünstigen Rahmenbedingungen, soweit es gelingt ihre Anreicherung und/oder ihr Wachstum am Ort der Verschmutzung hinreichend zu stimulieren. Aus dem neueren Schrifttum sei verwiesen auf die Veröffentlichungen in Chemische Industrie 5/91, 10 bis 12 "Hunger auf Erdöl" sowie in Erdöl und Kohle - Erdgas, 44 April 1991, 197 bis 200, Th. Höpner et al. "Die Ölkatastrophe im Persisch-Arabischen Golf" sowie die dort zitierte umfangreiche Literatur.

Das Arbeitsprinzip der Bioremediation sieht die optimale Förderung des Wachstums der Schmutz verzehrenden Mikroorganismenpopulationen vor. Zwei wichtige Hilfsmittel stehen hier im Vordergrund: Zum einen bedarf es der Zufuhr wachstumsfördernder Elemente, die in der Regel im Verschmutzungsbereich in nicht hinreichenden Konzentrationen zur Verfügung stehen. Hierbei handelt es sich in erster Linie um anorganische und/oder organische Verbindungen des Stickstoffs und des Phosphors, die als Nährstoffkonzentrate zur Stimulation und als Wachstumshilfe für die beschleunigte An- und Aufzucht der Kohlenwasserstoffverbindungen verzehrenden Mikroorganismen angeboten werden. Zum anderen werden häufig - insbesondere zur Beschleunigung des biologischen Abbaus in den Anfangsphasen - vorgebildete Konzentrate geeigneter Kohlenwasserstoff verzehrender Mikroorganismen eingesetzt, die beispielsweise im wöchentlichen Abstand auf die verschmutzten Bereiche aufgetragen werden können. Je nach der gegebenen Situation, insbesondere der individuellen Vorgeschichte der reinigungsbedürftigen Gebiete kann allerdings gegebenenfalls auch davon ausgegangen werden, daß ein solches Animpfen mit Mikroorganismen-Konzentraten unnötig ist. In der Regel wird das immer der Fall sein, wenn der natürliche Vorgang des biologischen Abbaus bereits zur Ausbildung hinreichender Konzentrationen von Mikroorganismenstämmen geführt hat, vgl. in diesem Zusammenhang beispielsweise die 2. der eingangs zitierten Literaturstellen.

Aus der GB-A-2065631 sind Mittel zur verbesserten mikrobiologischen Entfernung von Kohlenwasserstoffen aus verschmutztem Wasser bzw. verschmutztem Boden bekannt, wobei diese neben Tensiden und Alkoholen eine phosphorhaltige Komponente sowie als stickstoffhaltige Komponente Melaminderivate enthalten. Aus der GB-A-2115399 werden zur Entfernung von Ölrückständen Dispergiermittel vorgeschlagen, die neben Tensiden und einem Lösungsmittel mindestens ein Monoalkylguanidiniumsalz enthalten müssen. Ggf. kann als zusätzliche Nährstoffkomponente eine stickstoffhaltige Verbindung, beispielsweise Alkali- oder Erdalkalisalze von Phosphorsäureestern von Fettalkoholen enthalten sein. Aus der DE-OS-2739428 ist ein Verfahren zur Reinigung von Süß- und Seewasser von Rohöl- und Erdölprodukten bekannt, wobei dazu Verbindungen, die Phosphor und langsam freisetzbaren Stickstoff enthalten, verwendet werden. Als Phosphorquelle werden Lecitin oder Phosphatide synthetischen oder natürlichen Ursprungs genannt.

In der nicht vorveröffentlichten deutschen Patentanmeldung DE-P 41 31 714.9 wird bereits ein verbessertes Nährstoffgemisch für die Bioremediation verschmutzter Böden und Gewässer vorgeschlagen. Das dort beschriebene Gemisch liegt als lagerstabile Lösung oder Emulsion vor und enthält P- und N-Quellen in flüssiger Mischphase aus bioverträglichen wasserlöslichen Trägerkomponenten auf Glycerinbasis und bioverträglichen öllöslichen Trägerkomponenten auf Glycerinesterbasis. Wenngleich diese Anmeldung eine Lösung vorschlägt, bei der in die kontaminierte Bodenprobe nur solche Stoffe eingeführt werden, die biologisch gut abbaubar sind, so wird doch in Gestalt der Glycerinester in substantiellem Umfang eine weitere C-Quelle eingeführt. Unter ungünstigen Wuchsbedingungen kann es dabei vorkommen, daß durch Vorhandensein dieser Komponenten Mikroorganismen, die nicht zum Abbau von Schadstoffen geeignet sind, in ihrem Wachstum gefördert werden und sich zu Lasten der abbauenden Mikroorganismen vermehren.

Vor diesem Hintergrund ist es Aufgabe der Erfindung, ein Nährstoffgemisch bereitzustellen, das emulgierende Wirkung hat und dabei P- und N-lieferende Substanzen enthält, aber aus möglichst wenig reinen C-Verbindungen aufgebaut ist.

Dabei ist eine weitere Aufgabe der Erfindung, dieses Mittel so zu bereiten, daß es möglichst wenig Rückstände in der zu reinigenden Bodenprobe hinterläßt. Eine weitere Aufgabe der Erfindung liegt darin, ein Nährstoffgemisch bereitzustellen, das die schadstoffabbauenden Mikroorganismen gegenüber anderen Mikroorganismen bevorzugt.

### Gegenstand der Erfindung

Gegenstand der Erfindung ist dementsprechend in einer ersten Ausführungsform ein Nährstoffkonzentrat zur Stimulation und als Wachstumshilfe für die beschleunigte An- und Aufzucht von Kohlenwasserstoffverbindungen verzehrenden Mikroorganismen für deren Einsatz beim biologischen Abbau organischer Komponenten, enthaltend wasser- und/oder öllösliche Verbindungen des Phosphors (P) und des Stickstoffs (N), die in Abmischung mit weiteren wasser- und/oder öllöslichen organischen Mischungskomponenten vorliegen, denen wenigstens anteilsweise Nährstoffcharakter für das Mikroorganismenwachstum zukommt, dadurch gekennzeichnet, daß es als flüssige wasserbasierte Zubereitung vorliegt und einen Ester der Phosphorsäure als Emulgator und P-Quelle sowie 10 bis 40 Gew.-% Harnstoff als N-Quelle enthält.

Gegenstand der Erfindung ist weiterhin die Verwendung solcher Nährstoffmischungen in Kombination mit Konzentraten von Mikroorganismen, die zum Abbau von Kohlenwasserstoffverbindungen befähigt und dabei bevorzugt natürlichen Ursprungs sind. Als wichtige Einsatzgebiete sind hier zu nennen die Sanierung von Böden, von Süßwasser und/oder Salzwasser unter Beseitigung von Verschmutzungen auf Basis von Kohlenwasserstoffverbindungen, aber auch die Reinigung von Arbeitsgerätschaften, Leitungen, Großbehältern einschl. Tankschiffen und dergleichen, unter Verwendung der erfindungsgemäß definierten Arbeitsmittel.

In einer besonderen Ausführungsform betrifft die Erfindung die Verwendung der Nährstoffmischungen insbesondere in Kombination mit zum Kohlenwasserstoffabbau befähigten Mikroorganismen-Konzentraten zur Entsorgung von Öl-benetzten Cuttings aus land- oder seegestützten geologischen Bohrungen, beispielsweise aus dem Aufschluß von geologischen Wertstoffvorkommen.

### Einzelheiten der Erfindung

Im Rahmen der Erfindung kommt den eingesetzten Phosphorsäureestern besondere Bedeutung zu. Diese Verbindungen dienen zum einen als P-Quelle, zum anderen als Emulgatoren. Zwar wird die Wirkungsweise nicht voll verstanden, doch kann angenommen werden, daß sich die Verbindungen an der Grenzfläche zwischen dem hydrophoben Schadstoff und dem in der Bodenprobe vorhandenen Wasser ansammeln und dort einerseits emulgierend wirken, andererseits die Phosphorquelle in unmittelbarer Nachbarschaft zum Schadstoff verfügbar halten, so daß sie insbesondere für solche Mikroorganismen nutzbar wird, die zum Schadstoffabbau befähigt sind. Bevorzugte Phosphorsäureester sind solche, die von Mikroorganismen aufgenommen werden können. Unter den hier einsetzbaren Verbindungen kommen daher den Phospholipden besondere Bedeutung zu. Phosphorlipide sind amphiphile Substanzen, die aus pflanzlichen oder tierischen Zellen gewonnen werden. Bevorzugte Phospholipide sind die Glycerophospholipide, die üblicherweise auch als Lecithin bezeichnet werden. Weniger bevorzugt die Sphingophospholipide. Bekannte und einsetzbare Substanzen sind hier die Diacylphospholipide, Phosphatidylcholine, Phosphatidylethanolamine, Phosphatidylinositole, Phosphatidylserine, Phosphatidylglycerine, Phophatidylglycerinphosphate, Diphosphatidylglycerin, N-Acylphosphatidylethanolamin und Phosphatidinsäure. Bevorzugt sind die Monoacylphospholipide, Lysophosphatidylcholine, Lysophosphatidylethanolamine, Lysophosphatidylinositole, Lysophosphatidylserine, Lysophosphatidylglycerole, Lysophosphatidylglycerophosphate, Lysodiphosphatidylglycerine, Lyso-n-acylphosphatidylethanolamine und Lysophosphatidinsäure. Wegen der Zugänglichkeit wird der Fachmann in erster Linie zu technisch verfügbaren Phosphatidylglyceriden greifen, die als pflanzliche oder tierische Lecithine und Zephaline im Handel sind. Diese Zubereitungen werden meist aus Ölen, wie Maiskeimöl oder Baumollsaatöl oder Sojaöl gewonnen. Im Rahmen der Erfindung bevorzugt sind die enzymatisch hydrolisierten Glycerophospholipide (enzymatisch hydrolisiertes Lecithin), die aufgrund der Abspaltung eines Fettsäurerestes einen hydrophileren Charakter aufweisen. Ausgenommen sind dabei lediglich solche Produkte, die durch die enzymatische Hydrolyse ihren Phosphorsäurerest verloren haben.

Neben oder anstelle der genannten Phospholipide können als P-Quelle der erfindungsgemäßen Nährstoffkonzentrate auch Partialester der Phosphorsäure mit Fettalkoholen und dabei insbesondere entsprechende Partialester mit geradkettigen Fettalkoholen eingesetzt werden. Besonders geeignet sind hier die Ester kürzerkettiger Fettalkohole, also beispielsweise solcher mit C₆ bis C₁₀. Eingesetzt werden können jedoch auch Alkylphosphate mit längeren Fettalkoholresten, also C₁₂ bis C₂₄. Weiterhin eingesetzt werden können auch Fettalkoholetherphosphate. Es sind dies Phosphorsäurepartialester ethoxylierter Fettalkohole, wobei die Fettalkohole 8 bis 24 C-Atome aufweisen und mit einem bis 10 mol, vorzugsweise 4 bis 6 mol Ethylenoxid pro mol Fettalkohol ethoxyliert sind.

Die genannten Phosphorester werden in den erfindungsgemäßen Nährstoffkonzentraten in Mengen von 10 bis 40 Gew.-% und vorzugsweise in Mengen von 20 bis 30 Gew.-% eingesetzt.

Die erfindungsgemäßen Nährstoffkonzentrate enhalten weiterhin eine N-Quelle in Form anorganischer und/oder organisch gebundenen Stickstoffs. Bevorzugt sind N-Quellen, die nur organisch gebundenen Stickstoff enthalten, da der Einsatz anorganisch gebundenen Stickstoffs zu einer Versalzung des Bodens führen kann.

In Betracht kommen hier beispielsweise anorganische Salze wie Alkalinitrat oder -nitrite oder Ammoniumsalze, zum Beispiel Ammoniumsulfat oder insbesondere Ammoniumnitrat. Organische N-Quellen sind bioverträgliche organische Stickstoffverbindungen. Als besonders leicht zugänglich für die Steuerung und Begünstigung des Mikroorganismenwachstums hat sich im Rahmen der erfindungsgemäßen Lehre Harnstoff erwiesen, der in Mengen von etwa 10 bis 40 Gew.-%, insbesondere 15 bis 30 Gew.-% in den Nährstoffkonzentraten vorliegt. Dabei hat es sich gezeigt, daß bei der Verwendung von Harnstoff in den vorgenannten Konzentrationen ein Konservierungseffekt gegen nicht gewünschten mikrobiellen Befall erzielt wird, so daß die Nährstoffkonzentrate die erforderliche hohe Lagerstabilität aufweisen.

Nach einer besonderen Ausführungsform der Erfindung können die erfindungsgemäßen Nährstoffgemische ohne eine zusätzliche N-Quelle eingesetzt werden, wenn als P-Quelle Phospholipide verwendet werden, da diese Substanzen sowohl P als auch N enthalten.

Die erfindungsgemäßen Nährstoffkonzentrate können zur Unterstützung der emulgierenden Wirkung der Phosphorsäureester biologisch verträgliche Tenside (mit Ausnahme der in der älteren Anmeldung DE 41 31 714 beanspruchten Glycerinester) enthalten. Eingesetzt werden können hier anionische Tenside oder Mischungen aus anionischen Tensiden mit nichtionischen Tensiden, aber auch nichtionische Tenside allein. Geeignete anionische Tenside sind leicht biologisch abbaubare anionische Tenside, wie beispielsweise Seifen. Auch Alkylsulfate, insbesondere Fettalkoholsulfate sind einsetzbar. Weniger geeignet sind anionische Tenside auf petrochemischer Basis, wie beispielsweise Alkylbenzolsulfonat oder Alkylethersulfate. Bevorzugte nichtionische Tenside sind Alkylglykosidverbindungen, die bevorzugt hergestellt worden sind aus geradkettigen Fettalkoholen mit wenigstens 8 C-Atomen. Geeignete Alkylglykosidverbindungen enthalten beispielsweise Fettalkoholreste des Bereichs C₈ bis C₁₈, insbesondere C₁₀ bis C₁₈ und weisen einen durchschnittlichen Polymerisationsgrad der Oligoglykosidreste im Bereich von etwa 1,2 bis 5 auf. Entsprechende Alkylglykosidverbindungen mit einem DP-Wert von etwa 1,5 bis 5 können insbesondere im Bereich der HLB-Werte von etwa 10 bis 18 nicht nur als wirkungsvolle tensidische Zusatzstoffe vom O/W-Typ angesehen werden, sie zeichnen sich darüberhinaus durch eine besondere Bioverträglichkeit aus, die unter anderem durch ihre vergleichsweise rasche Abbaubarkeit mitbedingt ist.

Neben oder anstelle solcher Tenside können aber auch Biotenside biologischen Ursprungs mitverwendet werden, als Beispiele seien benannt Sophoroselipid, Trehaloselipid oder Lipopeptide, wie sie als Stoffwechselprodukte oder Membranbestandteile einer Mehrzahl von Mikroorganismenstämmen bekannt sind. In diesem Zusammenhang gilt für das erfindungsgemäße System die folgende Zusatzüberlegung: Das Vorliegen von Biotensiden kann nicht nur durch die Zusammensetzung des Nährstoffkonzentrats in einleitenden Verfahrensschritten der Remediation gewährleistet werden. Auch im Verlauf des mikrobiologischen Abbaus der Kohlenwasserstoffverbindungen wird die in situ-Bildung von Stoffwechselprodukten biotensidischen Charakters durch die Nährstoffgemische gefördert. Diese Biotenside wirken - ebenso wie die eingangs genannten Phosphorsäureester als Dispergatoren und Emulgatoren und beschleunigen somit den Ölabbau.

Neben oder anstelle der genannten nichtionischen Tenside können auch Sorbitanester eingesetzt werden, so beispielsweise Sorbitanmonostearat oder Sorbitanmonooleat. Gleichfalls einsetzbar, aber weniger zu empfehlen sind ethoxylierte Sorbitanester.

In den erfindungsgemäßen Nährstoffkonzentraten liegen die Tensidverbindungen der angegebenen Art üblicherweise in Mengen von 0,5 bis 5 Gew.-% vor. In der Regel reichen Tensidmengen bis etwa 1 Gew.-% aus, um die beabsichtigte Anstoßstimulation des Mikroorganismenwachstums auszulösen und zu begünstigen. So können gerade die Tenside auf Basis Alkylglykosidverbindungen etwa im Bereich von etwa 0,5 bis 1 Gew.-% - bezogen auf Gesamtmischung - zur wirkungsvollen Verbesserung des Mikroorganismenwachstums Verwendung finden.

Nährstoffkonzentrate der hier geschilderten Art sind lagerstabile Produkte, die zum Ausbringen auf feste und/oder flüssige Oberflächen zunächst mit einer mehrfachen Menge an Wasser vermischt werden. Geeignet ist beispielsweise die Aufmischung des Nährstoffkonzentrates mit der 5- bis 50fachen Gewichtsmenge an Wasser, wobei Mengen von etwa 15 bis 30 Gewichtsteilen Wasser auf 1 Gewichtsteil des Nährstoffkonzentrats bevorzugte Mischungsbereiche sind. Das Produkt kann dann in dieser wäßrigen Form auf die zu behandelnden Oberflächen aufgebracht werden. Dabei wird das Nährstoffkonzentrat in Mengen von vorzugsweise 5 bis 200 mg pro g Verunreinigung in die kontaminierte Bodenprobe eingearbeitet.
Besondere Vorteile des erfindungsgemäßen Nährstoffgemisches sind beispielsweise dessen vollständige Abbaubarkeit, die Tatsache daß die erfindungsgemäßen Stoffmischungen keine umweltschädlichen und/oder umweltstörenden Chemikalien enthalten, dabei vergleichsweise kostengünstige Stoffmischungen sind, die leicht und schnell auch großflächig anwendbar sind. Das Wachstum und die Leistungsfähigkeit der beispielsweise in ölverschmutzten Böden präsenten Mikroflora wird durch diese Mischung aus Nährstoffen, ausgewählten oleophilen und hydrophilen Trägerstoffen und Emulgatoren stark gefördert. Die Mitverwendung beschränkter Mengen von Emulgatoren von Anfang an erhöht die Bioverfügbarkeit der beispielsweise ölhaltigen Verschmutzung. Dadurch wird der Angriff der Mikroorganismen auf die Verschmutzung erleichtert und deren Abbau beschleunigt.

Häufig ist allerdings der Fall gegeben, daß unbehandelte ölverschmutzte Böden und/oder Wasserbereiche nur geringe Mengen an ölabbauenden Mikroorganismen aufweisen. Das hat zur Folge, daß der natürliche mikrobielle Abbau der Verschmutzung besonders in der Anfangsphase zu langsam vor sich geht. Hier sieht die Lehre der Erfindung vor, an die Verschmutzungen angepaßte Mikroorganismen-Konzentrate wenigstens zu Beginn der Reinigungsschritte zusammen mit den Nährstoffkonzentraten einzusetzen. Die Erfindung will dabei insbesondere nicht-pathogene harmlose Bakterienpopulationen verwenden, die bevorzugt aus der Umwelt isoliert und in einer getrennten Vorstufe angereichert worden sind. Dieses Animpfen der verschmutzten Bereiche unterstützt die natürlich vorhandene Mikroflora beim Abbau der Kohlenwasserstoffverbindungen. Dadurch wird in hohem Maße die Abbaurate - beispielsweise die Öl-Abbaurate - insbesondere in der kritischen Anfangsphase verbessert. Innerhalb weniger Tage nach der Applikation der eingangs aufgebrachten Hilfsstoffe reichem sich neben den Starterkulturen die natürlichen Keime an und setzen den Abbau fort. Zur Gewährleistung einer breiten Anwendbarkeit kann es zweckmäßig sein als Starterkulturen Mikroorganismenstämme einzusetzen, die auch salzwasserfest sind, so daß damit beispielsweise Ölverschmutzungen auch im Meeresbereich beziehungsweise an Meerwasserstränden wirkungsvoll bekämpft werden können.

In einer wichtigen Ausführungsform der Erfindung werden zusammen mit den Nährstoffkombinationen Mikroorganismen-Konzentrate eingesetzt, die durch getrenntes Anzüchten solcher natürlicher Stämme gewonnen worden sind, die aus Kohlenwasserstoff-verschmutzten Fundstellen natürlichen Ursprungs isoliert wurden. Dabei können Konzentrate entsprechender Mikroorganismenstämme bevorzugt sein, die ihrerseits Biotenside als Stoffwechselprodukte bilden. Ohne Anspruch auf Vollständigkeit seien einige mögliche Starterkulturen aufgezählt, die in der Regel allerdings nicht als isolierte Stämme sondern als Mischung einer Mehrzahl von Stämmen Verwendung finden: Pseudomonas oleovorans DSM 1045; Pseudomonas putida DSM 548 und DSM 50208; Acinetobacter calcoaceticus DSM 590; Nocardia paraffineus ACC 21198; Arthrobacter paraffineus ATCC 15591.

Die im Rahmen der Erfindung zum Einsatz kommenden Nährstoffgemische und die gegebenenfalls gleichzeitig damit einzusetzenden Mikroorganismenkulturen können ganz allgemein zur Sanierung von Böden, von Süßwasser und/oder von Salzwasser und damit zur Beseitigung und/oder zur Beschleunigung des Abbaus von Verschmutzungen auf Basis von Kohlenwasserstoffverbindungen auf Land und/oder Wasser zum Einsatz kommen. Das heute im großem Umfang in diesem Zusammenhang relevante Einsatzgebiet ist die Bekämpfung von unerwünschten Ölverschmutzungen, insbesondere Rohölverschmutzungen oder die Beseitigung von entsprechenden Altlasten in üblicherweise räumlich eingegrenzten Bodenbereichen. Die erfindungsgemäße Lehre eignet sich aber auch zur Anwendung in ganz anderer Weise: So können durch Bioremediation ölverschmutzte Arbeitsgerätschaften, beispielsweise Leitungen oder Großbehälter bis hin zu Tankschiffen, wirkungsvoll gereinigt werden. Bezüglich der jeweils einzusetzenden Technologie wird auf den einschlägigen druckschriftlichen Stand der Technik verwiesen, s. hierzu beispielsweise Al W. Bourquin "Bioremediation of Hazardous Waste" in "Biofuture" September 1990, 24 bis 35.

In einer besonders wichtigen Ausführungsform betrifft die Erfindung die Verwendung der beschriebenen Nährstoffmischungen - insbesondere in Kombination mit zum Kohlenwasserstoffabbau befähigten Mikroorganismen-Konzentraten - zur Entsorgung von Öl-benetzten Cuttings aus land- oder seegestützten geologischen Bohrungen, beispielsweise aus dem Aufschluß von geologischen Wertstoffvorkommen. Im einzelnen liegt hier der folgende Sachverhalt vor: Im Rahmen der Erschließung von beispielsweise Erdöl und/oder Erdgas werden heute in großem Umfange unter Normalbedingungen fließfähige Bohrlochbehandlungsmittel auf Basis einer geschlossenen Ölphase eingesetzt. Ein charakteristisches Beispiel hierfür sind Bohrspülflüssigkeiten und darauf aufgebaute Bohrspülschlämme vom W/O-Typ. Andere Beispiele sind die sogenannten Spotting fluids, Spacer, Hilfsflüssigkeiten für work-over und Stimulierung und für das Fracturing.

Bohrlochbehandlungsmittel mit geschlossener Ölphase wurden in der Praxis bisher nahezu ausschließlich durch Mineralölfraktionen gebildet. Damit ist eine nicht unbeträchtliche Belastung der Umwelt verbunden, wenn beispielsweise die Bohrschlämme unmittelbar oder über das erbohrte Gestein in die Umwelt gelangen. Mineralöle sind ohne zusätzliche Hilfsmaßnahmen nur schwer und anerob praktisch nicht abbaubar und damit als langfristige Verschmutzung anzusehen.

Aus jüngerer Zeit bestehen einige Vorschläge zur Minderung dieser Problematik. So werden in einer größeren Zahl von Anmeldungen Vorschläge zum Austausch der Mineralölfraktionen gegen ökologisch verträgliche leicht abbaubare Ölphasen. Dabei werden unterschiedliche Typen von Austauschölen dargestellt, die auch in Mischung miteinander eingesetzt werden können. Es handelt sich hierbei um ausgewählte oleophile Carbonsäureester, um oleophile Diester der Kohlensäure, um wenigstens weitgehend wasserunlösliche und unter Arbeitsbedingungen fließfähige oleophile Alkohole und um entsprechende Ether. Summarisch wird hier verwiesen auf die Veröffentlichungen beziehungsweise älteren Anmeldungen DE 38 42 659, 38 42 703, 39 07 391, 39 07 392, 39 03 785, 39 03 784, 39 11 238, 39 11 299 und 40 18 228.

Die erfindungsgemäße Lehre zur Stimulierung des mikrobiellen Abbaus von Kohlenwasserstoff-basierten Komponenten und insbesondere entsprechenden Ölen kann mit besonderem Vorteil eingesetzt werden zur raschen Beseitigung von Ölverschmutzungen beziehungsweise -rückständen, beispielsweise auf Bohrklein, wie sie im Rahmen des Arbeitens mit solchen Öl-basierten Systemen in der Praxis anfallen. Die Entsorgung der Öl-benetzten Cuttings, beispielsweise aus einer landgestützten Bohrung ist nach bisheriger Praxis nur durch Ablagerung auf einer Sondermülldeponie möglich. Es ist bekannt, daß der zur Verfügung stehende Deponieraum heute nur noch begrenzt ist. Vorgeschlagen sind dementsprechend vergleichsweise komplizierte tensidische Waschverfahren zur Reinigung und damit erleichterten Deponierung solcher Rückstandsprodukte aus der Bohrung. Die Lehre der Erfindung sieht den beschleunigten mikrobiellen Abbau der Ölrückstände durch Einsatz der Nährstoffkonzentrate, gegebenenfalls unter zusätzlicher Mitverwendung von Starterkulturen geeigneter Mikroorganismen vor. Die erfindungsgemäße Lehre ermöglicht beispielsweise die rasche Beseitigung von Öl-basierten Bohrschlämmen, die zusammen mit dem erbohrten Gestein aus dem Bohrloch ausgetragen und durch konventionelle Trennverfahren - beispielsweise Sieben - nicht von dem Bohrklein abgetrennt werden können. Durch Rückführung eines Anteils der sich im Rahmen einer solchen Aufarbeitung ausbildenden Mikroorganismenpopulation kann ein in verbesserter Weise deponiefähiges Bohrklein geschaffen werden.

Im praktischen Einsatz werden die eingangs geschilderten Nährstoffkonzentrate beispielsweise mit Wasser 1:20 verdünnt. Anschließend kann bei Bedarf die mitverwendete Starterkultur ausgewählter Mikroorganismen eingemischt werden. Die Mischung der Komponenten sollte möglichst unmittelbar vor dem Ausbringen der gebrauchsfertigen Mischung erfolgen, um die maximale Wirksamkeit der Produkte zu gewährleisten. Die gebrauchsfertigen Gemische können beispielsweise durch Versprühen kleinflächig oder auch großflächig ausgebracht werden. Es kann zweckmäßig sein, die Applikation jeweils im Wochenrythmus zu wiederholen.

Die Nachdosierung des Nährstoffkonzentrates kann in einfacher Weise überwacht werden. So kann man zu Beginn der Maßnahme sowohl die Harnstoff- als auch die Ammoniumionen-Konzentration in der zu behandelnden Probe bestimmen. Die Bildung von Ammoniumionen zeigt dann die Stoffwechselaktivität der Mikroorganismen bei der Verwertung von Harnstoff. Daher kann die Nachdosierung der Nährstoffe über den Ammoniumionengehalt bestimmt werden, d. h., wenn Harnstoff verbraucht ist, kann gewünschtenfalls weiterer zugefügt werden.

### Beispiele

Es wurde ein Nährstoffkonzentrat hergestellt aus:
Glycerophospholipid (enzymatisch hydrolisiertes Lecithin, Handelsname: Lipotin ^{(R}) NE der Firma Lucas Meyer) 20 Gew.-%
Harnstoff 20 Gew.-%
   Alkylpolyglykosid (C-Kettenlänge: C₈ bis C₁₆, D.P.: ca. 1,4), 2 Gew.-% sowie
Wasser ad 100 Gew.-%. Simuliert wurde die Reinigung von kontaminiertem Sand, und zwar unter folgenden Bedingungen:

### Versuchsansatz: Simulation der großtechnisch angewandten Sanierungstechnik "Biobeet"

- Substrat:: 2 kg Sand
- Verunreinigung:: Rohöl, 7 000 ppm
- Inkubationstemperatur:: 16 °C
- Beobachtungszeitraum:: 42 Tage
- Wirkstoffbehandlungsintervall:: 0,4 g Nährstoff-Konzentrat/kg Sand alle 10 Tage
- Schadstoffanalysenmethode:: DIN 38409 H18

### Ergebnis

| Inkubationzeit (Tage) | Restöl ohne Behandlung (%) | Restöl nach Nährstoff-Behandlung (%) |
|---|---|---|
| 0 | 100 | 100 |
| 14 | 68 | 36 |
| 29 | 64 | 33 |
| 35 | 54 | 27 |
| 42 | 52 | 20 |
| Anzahl der "Abbauspezialisten" nach 20 Tagen | <10 % | >70 % |

Die Schadstoffanalysenmethode nach DIN 38409 H18 besteht darin, daß man in einem organischen Lösungsmittelextrakt das spezifische spektrale Absorptionsmaß von Kohlenwasserstoffgemischen mittels Infrarotspektroskopie ermittelt. Die Anzahl der "Abbauspezialisten", d. h. der zum Abbau der Verunreinigung befähigten Mikroorganismen wurde bestimmt, indem man zunächst die Gesamtzahl der wachsenden oder wachstumsfähigen Mikroorganismen bestimmt und aus diesen die Anzahl der Abbauspezialisten ermittelt, indem man die Kulturen auf eine Nährstoffplatte überstempelt, die Hexadecan als einzige C-Quelle enthält.

Aber Verhalten unter maringen Bedingungen:

### Versuchsansatz: Simulation "Strandsanierung"

- Substrat:: 1 kg Meersand, Einstellen der Bodenfeuchte mit Meerwasser
- Verunreinigung:: 20 000 ppm gealtertes Rohöl ohne flüchtige Bestandteile
- Inkubationstemperatur:: 15 °C
- Beobachtungszeitraum:: 42 Tage
- Wirkstoffbehandlungsintervall:: 0,4 g Nährstoff-Konzentrat/kg Sand alle 10 Tage
- Schadstoffanalysenmethode:: Gaschromatographie

### Ergebnis:

| Inkubationszeit (Tage) | Restöl ohne Behandlung (%) | Restöl-Fraktionen nach Nährstoffbehandlung (%) | | |
|---|---|---|---|---|
| | | C17 | C18 | C20 |
| 0 | 100 | 100 | 100 | 100 |
| 7 | 98 | 90 | 93 | 86 |
| 14 | 98 | 76 | 87 | 72 |
| 30 | 99 | 58 | 68 | 58 |
| 42 | 98 | 45 | 54 | 41 |

### Ergebnis:

Das Nährstoffkonzentrat aktiviert das Abbaupotential der marinen Flora.

## Patentansprüche

1. Nährstoffkonzentrat zur Stimulation und als Wachstumshilfe für die beschleunigte An- und Aufzucht von Kohlenwasserstoffverbindungen verzehrenden Mikroorganismen für deren Einsatz beim biologischen Abbau organischer Komponenten, enthaltend wasser- und/oder öllösliche Verbindungen des Phosphors (P) und des Stickstoffs (N), die in Abmischung mit weiteren wasser- und/oder öllöslichen organischen Mischungskomponenten vorliegen, denen wenigstens anteilsweise Nährstoffcharakter für das Mikroorganismenwachstum zukommt, dadurch gekennzeichnet, daß es als flüssige wasserbasierte Zubereitung vorliegt und einen Ester der Phosphorsäure als Emulgator und P-Quelle sowie 10 bis 40 Gew.-% Harnstoff als N-Quelle enthält.

2. Nährstoffkonzentrat nach Anspruch 1, dadurch gekennzeichnet, daß es bioverträgliche Tenside mit Ausnahme von Glycerinestern als weitere Komponente enthält, vorzugsweise jedoch nichtionische Tenside.

3. Nährstoffkonzentrat nach den Ansprüchen 1 und 2, dadurch gekennzeichnet, daß es enthält
10 bis 40 Gew.-% Phosphorsäureester als Emulgator,
10 bis 40 Gew.-% Harnstoff und
Wasser ad 100 Gew.-%.

4. Nährstoffkonzentrat nach den Ansprüchen 1 bis 3, dadurch gekennzeichnet, daß es enthält
20 bis 30 Gew.-% Phosphorsäureester als Emulgator,
15 bis 30 Gew.-% Harnstoff und
0,5 bis 5 Gew.-% nichtionische Tenside und
Wasser ad 100 Gew.-%.

5. Nährstoffkonzentrat nach den Ansprüchen 1 bis 4, dadurch gekennzeichnet, daß es als Phosphorsäureester Phospholipide, Alkylphosphate und/oder Alkyletherphosphate enthält.

6. Nährstoffkonzentrat nach den Ansprüchen 1 bis 5, dadurch gekennzeichnet, daß es als Phosphorsäureester Glycerophospholipide enthält.

7. Nährstoffkonzentrate nach den Ansprüchen 1 bis 6, dadurch gekennzeichnet, daß es als Phosphorsäureester enzymatische, P-haltige Spaltprodukte von Glycerophospholipiden enthält.

8. Nährstoffkonzentrat nach den Ansprüchen 1 bis 7, dadurch gekennzeichnet, daß es als nichtionische Tenside Alkylglykosidverbindungen, hergestellt aus geradkettigen Fettalkoholen mit 8 bis 24 C-Atomen und Mono- und/oder Oligoglykosiden; Zuckerpartialester von Monocarbonsäuren mit 8 bis 24 C-Atomen, Sorbitanester, Biotenside biologischen Ursprungs wie Sophoroselipid, Trehaloselipid und Lipopeptide enthält.

9. Verfahren zur Reinigung verschmutzter Böden oder Gewässer, dadurch gekennzeichnet, daß man ein Nährstoffkonzentrat nach den Ansprüchen 1 bis 8 mit Wasser auf das 10- bis 50fache verdünnt und ein- oder mehrfach in einer Menge von 5 mg bis 200 mg pro g Verunreinigung in die kontaminierte Bodenprobe einarbeitet.

10. Verwendung der Nährstoffmischungen nach den Ansprüchen 1 bis 9, in Kombination mit Konzentraten von Mikroorganismen, die zum Abbau von Kohlenwasserstoffverbindungen befähigt und dabei natürlichen Ursprungs sind.

11. Ausführungsform nach Anspruch 10, dadurch gekennzeichnet, daß Mikroorganismen-Konzentrate eingesetzt werden, die aus Kohlenwasserstoff-verschmutzten Fundstellen natürlichen Ursprungs isoliert worden sind.

12. Ausführungsform nach den Ansprüchen 10 und 11, dadurch gekennzeichnet, daß Konzentrate von Mikroorganismenstämmen, die Biotenside als Stoffwechselprodukte bilden, eingesetzt werden.

13. Ausführungsform nach den Ansprüchen 10 bis 12, dadurch gekennzeichnet, daß die Nährstoffgemische zur Sanierung von Böden, Süßwasser und/oder Salzwasser, insbesondere zur Beseitigung und/oder zur Beschleunigung des Abbaus von Ölverschmutzungen und/oder anderen Verschmutzungen auf Basis von Kohlenwasserstoffverbindungen auf Land und/oder Wasser beziehungsweise zur Reinigung von Arbeitsgerätschaften, Leitungen, Großbehältern einschließlich Tankschiffen und dergleichen verwendet werden.

14. Verwendung der Nährstoffmischungen nach den Ansprüchen 10 bis 13 in Kombination mit zum Kohlenwasserstoffabbau befähigten Mikroorganismen-Konzentraten zur Entsorgung von Öl-benetzten Cuttings aus land- oder seegestützten geologischen Bohrungen, beispielsweise aus dem Aufschluß von geologischen Wertstoffvorkommen.

15. Ausführungsform nach Anspruch 14, dadurch gekennzeichnet, daß die Wirkstoffmischungen zur biologischen Entsorgung von Invert-Bohrschlämmen auf Basis oleophiler Carbonsäureester, oleophiler Kohlensäureester, oleophiler Ether und/oder oleophiler Alkohole verwendet werden.

## Claims

1. A nutrient concentrate for stimulation and as a growth aid for the accelerated cultivation and growth of hydrocarbon-consuming microorganisms for their use in the biological degradation of organic components, containing water-soluble and/or oil-soluble compounds of phosphorus (P) and nitrogen (N) present in admixture with other water-soluble and/or oil-soluble organic mixture components which are at least partly endowed with nutrient character for the growth of the microorganisms, characterized in that it is in the form of a liquid water-based preparation and contains an ester of phosphoric acid as emulsifier and P-source and 10 to 40% by weight of urea as N-source.

2. A nutrient concentrate as claimed in claim 1, characterized in that it contains biocompatible surfactants except for glycerol esters as a further component, but preferably nonionic surfactants.

3. A nutrient concentrate as claimed in claims 1 and 2, characterized in that it contains
10 to 40% by weight of phosphoric acid ester as emulsifier,
10 to 40% by weight of urea and
water to 100% by weight.

4. A nutrient concentrate as claimed in claims 1 to 3, characterized in that it contains
20 to 30% by weight of phosphoric acid ester as emulsifier,
15 to 30% by weight of urea and
0.5 to 5% by weight of nonionic surfactants and
water to 100% by weight.

5. A nutrient concentrate as claimed in claims 1 to 4, characterized in that it contains phospholipids, alkyl phosphates and/or alkylether phosphates as the phosphoric acid ester.

6. A nutrient concentrate as claimed in claims 1 to 5, characterized in that it contains glycerophospholipids as the phosphoric acid ester.

7. A nutrient concentrate as claimed in claims 1 to 6, characterized in that it contains P-containing hydrolysis products of glycerophospholipids as the phosphoric acid esters.

8. A nutrient concentrate as claimed in claims 1 to 7, characterized in that it contains alkyl glycoside compounds prepared from preferably linear fatty alcohols containing 8 to 24 carbon atoms and mono- and/or oligoglycosides; partial sugar esters of C₈₋₂₄ monocarboxylic acids, sorbitan esters, biosurfactants of biological origin, such as sophorose lipid, trehalose lipid and lipopeptides, as nonionic surfactants.

9. A process for the remediation of polluted soils or waters, characterized in that a nutrient concentrate of the type claimed in claims 1 to 8 is diluted with water to a volume of 10- to 50-fold and is incorporated in the contaminated soil sample one or more times in a quantity of 5 mg to 200 mg per g of pollution.

10. The use of the nutrient mixtures claimed in claims 1 to 9 in combination with concentrates of microorganisms which are capable of degrading hydrocarbon compounds and which are preferably of natural origin.

11. The use claimed in claim 10, characterized in that microorganism concentrates which have been isolated from hydrocarbon-contaminated localities of natural origin are used.

12. The use claimed in claims 10 and 11, characterized in that concentrates of microorganism strains which form biosurfactants as metabolism products are used.

13. The use claimed in claims 10 to 12, characterized in that the nutrient mixtures are used for the remediation of soils, fresh water and/or salt water, more particularly for the elimination and/or accelerated degradation of oil contamination and/or other contamination based on hydrocarbon compounds on land and/or water and for the cleaning of working equipment, pipes, large vessels, including marine tankers and the like.

14. The use of the nutrient mixtures claimed in claims 10 to 13 in combination with microorganism concentrates capable of degrading hydrocarbons for the disposal of oil-wetted cuttings from biological land-supported or offshore drilling, for example from the development of valuable geological occurrences.

15. The use claimed in claim 14, characterized in that the nutrient mixtures are used for the biological disposal of invert drilling muds based on oleophilic carboxylic acid esters, oleophilic carbonic acid esters, oleophilic ethers and/or oleophilic alcohols.

## Revendications

1. Concentré de substance nutritive, en vue de la stimulation et comme substance de croissance, pour la mise en culture et la reproduction de micro-organismes qui consomment des composés hydrocarbonés pour leur utilisation dans la dégradation biologique de composés organiques, contenant des composée de phosphore (P) et de l'azote (N), solubles dans l'eau et/ou solubles dans l'huile, mélangés avec d'autres composants de mélanges organiques solubles dans l'eau et/ou solubles dans l'huile, auxquels revient le caractère de substance nutritive pour la croissance de microorganismes, au moins partiellement,
caractérisé en ce qu'
- il se présente sous forme de préparation liquide, à base d'eau, et
- il renferme un ester de l'acide phosphorique en tant qu'agent émulsionnant comme sources de P, ainsi que de 10 à 40 % on poids d'urée comme source de N.

2. Concentré de substance nutritive selon la revendication 1,
caractérisé en ce qu'
il contient des agents tensioactifs bio-compatibles à l'exception d'esters de glycérol comme autre composant, de préférence cependant des agents tensioactifs non ioniques.

3. Concentré de substance nutritive selon les revendications 1 et 2,
caractérisé en ce qu'
il contient :
- de 10 à 40 % en poids d'ester d'acide phosphorique comme agent émulsionnant,
- de 10 à 40 % en poids d'urée, et
- de l'eau qsp 100 % en poids.

4. Concentré de substance nutritive selon les revendications 1 à 3,
caractérisé en ce qu'
il contient :
- de 20 à 30 % en poids d'ester d'acide phosphorique comme agent émulsionnant,
- de 15 à 30 % en poids d'urée, et
- de 0,5 à 5 % en poids d'agent tensioactif non ionique, et
- de l'eau qsp 100 % en poids.

5. Concentré de substance nutritive selon les revendications 1 à 4,
caractérisé en ce que
comme ester d'acide phosphorique il contient des phospholipides, des phosphates d'alkyle et/ou des phosphates d'alkyléther.

6. Concentré de substance nutritive selon les revendications 1 à 5,
caractérisé en ce qu'
il contient comme ester d'acide phosphorique des glycérephospholipides.

7. Concentré de substance nutritive selon les revendications 1 à 6,
caractérisé en ce qu'
il contient comme ester d'acide phosphorique des produits enzymatiques, contenant du P de coupure de glycérophospholipides.

8. Concentré de substance nutritive selon les revendications 1 à 7,
caractérisé en ce qu'
il contient comme agent tensioactif non ionique, des composés d'alkylglycoside produits à partir d'alcools gras à chaîne droite ayant de 8 à 24 atomes de carbone, et de mono- et/ou oligo-glycosides ; des esters partiels de sucre à partir d'acides monocarboxyliques ayant de 8 à 24 atomes de carbone, un ester de sorbitane, des bio-tensioactifs d'origine biologique comme un lipide de sophorose, un lipide de tréhalose et un lipopeptide.

9. Procédé d'épuration ses sols et des eaux,
caractérise en ce qu'
- on dilue un concentré de substance nutritive selon les revendications 1 à 8, avec de l'eau de 10 à 50 fois, et
- on l'incorpore en une ou plusieurs fois, en quantité de 5 mg à 200 mg par g d'impureté dans l'échantillon du sol contaminé.

10. Utilisation des mélanges de substances nutritives selon les revendications 1 à 9,
en combinaison avec des concentrés de micro-organismes qui sont aptes à la dégradation de composés hydrocarbonés et pour cela sont d'origine naturelle.

11. Mode d'exécution selon la revendication 10,
caractérisé en ce qu'
on met en oeuvre des concentrés de micro-organismes qui ont été isolés à partir d'emplacements de découverte contaminés par des hydrocarbures, d'origine naturelle.

12. Mode d'exécution selon les revendications 10 et 11,
caractérisé en ce qu'
on met en oeuvre des concentrés de souches de micro-organismes qui forment des bio-tensioactifs comme produits du métabolisme.

13. Mode d'exécution selon les revendications 10 à 12,
caractérisé en ce qu'
on utilise les mélanges de substances nutritives pour l'assainissement des sols, de l'eau douce, et/ou salée, on particulier pour l'élimination et/ou pour l'accélération de la dégradation de contaminations par l'huile et/ou d'autres contaminations à base de composée hydrocarbonés sur la terre et/ou sur l'eau, ou pour le nettoyage d'ustensiles de travail, de conduits, de réservoirs, y compris les bateaux-citernes et similaires.

14. Utilisation des mélanges de substances nutritives selon les revendications 10 à 13
en combinaison avec les concentrés de micro-organismes aptes à la dégradation des hydrocarbures, en vue de l'élimination de 〈〈 coupes 〉〉 (cuttings) souillées par de l'huile, provenant de forages géologiques effectués sur terre ou dans les mers, par exemple à partir de la dégradation de gisements de substances de valeur, géologiques.

15. Mode d'exécution selon la revendication 14,
caractérisé en ce que
les mélanges de substances actives utilisés en vue de l'élimination biologique de boues de forage inverses sont à base d'ester d'acide carboxylique oléophile, d'ester d'acide carbonique oléophile, d'éther oléophile, et/ou d'alcools oléophiles.
